Europäisches Patentamt

European Patent Office

Office européen des brevets

(19)

(11) Veröffentlichungsnummer: **0 305 777**
**A2**

(12) **EUROPÄISCHE PATENTANMELDUNG**

(21) Anmeldenummer: 88112961.3

(22) Anmeldetag: **10.08.88**

(51) Int. Cl.⁴: **C12N 15/00 , A61K 39/21 , A61K 39/42 , G01N 33/569 , C12P 21/00**

---

Patentansprüche für folgenden Vertragsstaat: ES.

(30) Priorität: **14.08.87 DE 3727137**

(43) Veröffentlichungstag der Anmeldung:
**08.03.89 Patentblatt 89/10**

(84) Benannte Vertragsstaaten:
**AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Bröker, Michael, Dr.**
**Lindenweg 16**
**D-3550 Marburg(DE)**
Erfinder: **Krupka, Udo, Dr.**
**Oberer Eichweg 29**
**D-3550 Marburg(DE)**
Erfinder: **Abel, Karl-Josef, Dr.**
**Am Ziegenberg 6**
**D-3550 Marburg(DE)**

(74) Vertreter: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

---

(54) **Herstellung von Antigenen des Human Immunodeficiency Virus Typ 1 sowie deren Verwendung als diagnostisches Mittel und Therapeutikum.**

(57) Die Herstellung von unprozessierten HIV-1 Hüll- und core-Proteinen durch Expression in E. coli wird beschrieben. Dabei wirkt die Fusion mit dem Amino-terminalen Ende von E. coli β-Galactosidase schützend vor proteolytischem Abbau der Fusionsproteine. Die so erhaltenen HIV-1 Proteine lassen sich als Diagnostikum oder als Heilmittel verwenden.

FIG.1

EP 0 305 777 A2

## Herstellung von Antigenen des Human Immunodeficiency Virus Typ 1 sowie deren Verwendung als diagnostisches Mittel und Therapeutikum

Das erworbene Immunmangelsyndrom (Acquired Immune Deficiency Syndrome, AIDS) hat als neues Krankheitsbild seit 1979 zunehmende Aufmerksamkeit gefunden.

Als Erreger konnte von Montagnier und Mitarbeitern ein Virus isoliert und weitgehend charakterisiert werden, das sie als Lymphadenopathie-assoziiertes Virus (LAV-I) bezeichneten. Kurz darauf beschrieben Gallo und Mitarbeiter ein ähnliches pathogenes Virus unter der Bezeichnung Humanes T-Zellen-lymphotrophes Virus III (HTLVIII). Ein drittes Isolat wurde "AIDS-assoziiertes Retrovirus" (ARV) benannt. Im Mai 1986 schlug ein Unterkommittee des International Committee on the Taxonomy of Viruses (ICTV) vor, die inzwischen weitgehend akzeptierte Bezeichnung Human Immuno-deficiency Virus (HIV) für den Erreger von AIDS zu verwenden, das in der Folge in "HIV-1" präzisiert wurde. Das HIV-1 zeigt in seiner genetischen Organisation eine enge Verwandtschaft mit den Lentiviren, den Erregern der Visna-Maedi-Krankheit der Schafe, der Ziegenenzephalitis und der infektiösen Anämie der Pferde. Schätzungen über die Zahl der infizierten Nordamerikaner liegen bei 1,5 bis 3,0 Millionen. Die Letalität der vollmanifestierten Erkrankung wird im allgemeinen mit 50 % und darüber angegeben. Es muß mit durchschnittlichen Inkubationszeiten von über fünf Jahren gerechnet werden, wobei als Zeitraum bis zur klinischen Manifestation auch durchaus 15 bis 20 Jahre diskutiert werden.

Das HIV-1 ist ein Retrovirus und hat einen Durchmesser von ca. 100 nm, ist von einer Lipidhülle umgeben und trägt auf diese Hülle dichtstehende, knopfförmige Glykoproteinfortsätze (Knobs). Diese Knobs vermitteln über ihre Rezeptorspezifität bei der Infektion den Kontakt zur Wirtszelle und werden als Virusoberflächenstruktur als Ziel der humoralen Abwehr des infizierten Organismus angesehen. Die Knobs bestehen aus dem Glykoprotein gp120, wobei dieses Glykoprotein über das Transmembranglykoprotein gp41 an die Virushülle gebunden ist. Die Vorläuferform von gp120 und gp41 ist ein gemeinsames Protein, das gp160, das durch eine viruskodierte Protease spezifisch in die beiden Endformen überführt wird. Direkt unter der Lipidhülle befindet sich das Protein p17 und an der Peripherie der tubulären Corehülle das p24-Protein. Das basische Protein p15 scheint in enger Assoziation mit dem Erbmaterial, das aus einzelständiger RNA besteht, vorzuliegen. p17, p24 und p15 sind Spaltprodukte eines gemeinsamen Vorläufers von ca. 55 000 Dal (precursor). Als weiteres charakteristisches Merkmal findet sich in der Nähe der RNA auch die viruskodierte RNA-abhängige DNA-Polymerase (Reverse Transkriptase). Diese hier beschriebenen HIV-1-Proteine induzieren im infizierten Wirt alle mehr oder weniger die Produktion von spezifischen Antikörpern.

Der Nachweis von Antikörpern gegen HIV-1 weist auf eine stattgefundene Exposition des Organismus mit dem Virus und damit auf einen möglichen Virusträger hin. Deshalb ist diese Bestimmung für die Diagnostik entsprechender Krankheitsbilder, für epidemiologische Untersuchungen von Risikogruppen und von Blutspendern von entscheidender Bedeutung.

Die übliche Diagnose von Patientenserum auf anti-HIV-1 Antikörper wird durch ELISA- und Western Blot-Techniken durchgeführt, wobei nach in vitro-Kultur des Virus und anschließender Isolierung angereicherte Proteine des Virus als Antigen verwendet werden. Seren von AIDS-Patienten reagieren in der Western Blot-Analyse vorwiegend mit den Hüllproteinen gp120, gp41 und gp160 (precursor) und dem core Protein p24 und p55 (precursor), aber auch mit anderen viralen Proteinen, wie der Reversen Transkriptase (p53/p65). Die Leistungsfähigkeit der Antikörper-Teste, die auf der Verwendung von klassisch gewonnenem Antigenmaterial beruhen (Teste der 1. Generation), ist im allgemeinen durch zwei Nachteile eingeschränkt. Einerseits enthalten die Virusproteinpräparationen, bedingt durch die Verwendung humaner Zellinien für die Virusanzucht nicht unerhebliche Kontaminationen der Wirtszelle, an die wiederum in der Probe vorhandene Antikörper binden, welche im Verlauf von Autoimmunkrankheiten (antinukleäre Antikörper, antimitochondriale Antikörper u.ä.) oder nach Polytransfusionen (humanes Lymphozyten-Antigen) gebildet werden. Bei der vornehmlich angewendeten indirekten Antikörperbestimmung werden demzufolge auch Nicht-HIV-1-spezifische Antikörper erfaßt (bei Verwendung von Antihuman IgG-Konjugat), was zu falsch-positiven Resultaten führt. Andererseits sind aber bei solch einem Testprinzip auch falsch-negative Ergebnisse zu erwarten. Dies beruht darauf, daß das gp120 bei der Aufarbeitung des HIV-1 sich leicht vom Viruspartikel ablöst und als Antigen in der Präparation gar nicht mehr oder nur noch in Spuren vorhanden ist. Zudem ist bei dem indirekten Test durch die Notwendigkeit von Vorverdünnungen die Handhabbarkeit nicht befriedigend und die Verwechslungsgefahr nicht zu vernachlässigen.

Da das gesamte Genom des HIV-1 als cDNA vorliegt (Ratner et al., Nature 313, 277-284 (1985)), wurden deshalb von verschiedenen Seiten Versuche unternommen, einzelne Proteinkomponenten des HIV durch gentechnologische Methoden herzustellen. Spezifische Anteile der Hüllproteine gp120 und gp41 wurden bereits in E.coli exprimiert (Cabradilla et al., Biotechnology 4,128 -133 (1986); Crowl et al., Cell 41, 979-986 (1985); Chang et al., Science 228, 93-96 (1985); Kiyokawa el al., Proc, Natl. Acad. Sci. USA 81, 6202-6206 (1984); Change et al., Biotechnology 3, 905-909 (1985); Schulz et al., Lancet, 111-112 (1986)). Es zeigte sich allerdings, daß ein großer Teil dieser rekombinanten gp120 bzw. gp41 Antigene unter den dort beschriebenen Expressionsbedingungen instabil ist und nicht oder nur in sehr geringen Mengen isoliert werden kann. Außerdem sind die jeweils exprimierten Anteile der Hüllproteine im Hinblick auf das jeweilige Hüllprotein recht klein, so daß aufgrund der genetischen Heterogenität des HIV-1 nicht ausgeschlossen werden kann, daß Patientenseren diese rekombinanten HIV-Antigene nicht oder nur mäßig erkennen. Daher ist es zweckmäßiger, einen möglichst großen Anteil des gp160 als Antigen zu exprimieren, um möglichst viele Epitope abzudecken. Als Alternative bietet sich an, kleinere gp160-Anteile, für die gezeigt werden konnte, daß sie relevante Epitope tragen, herzustellen. Ähnlich verhält sich die Situation mit in E.coli hergestellten core-Anteilen (Ghrayeb et al., DNA, 5, 93-99 (1986); Steiner et al., Virology 150, 283-290 (1986)). Patienterseren reagieren mit rekombinanten p24-Antigenen, doch ist inzwischen sichergestellt, daß p24 als Antigen im Testsystem allein nicht ausreicht.

Aus diesen Gründen wurde angestrebt, möglichst große Anteile der Hüllproteine und der core-Antigene in Mikroorganismen zu exprimieren.

Bei der Produktion von HIV-1 Hüllproteinen in eukaryotischen Zellsystemen ist davon auszugehen, daß das Vorläuferprotein gp160 in gp120 und gp 41 gespalten wird. Es ist möglich, daß die Aminosäuresequenz, die innerhalb dieser Proteasespaltstelle liegt, Teil eines wichtigen Epitops ist. Weiterhin ist denkbar, daß durch Spaltung des gp160 und folglich veränderter Sekundär- und Tertiärstruktur, Epitope im Übergangsbereich von gp120 und gp 41 verlorengehen. Eine Expression von HIV-1 Hüllprotein in E. coli würde solch eine spezifische Proteolyse des gp160 unwahrscheinlich machen, so daß deshalb die Expression von HIV-1 Antigenen in E. Coli vorgenommen wurde.

Zusätzlich sollte die Produktion der Antigene möglichst auf demselben Expressionssystem basieren und eine einfache Reinigung der rekombinanten Virusantigene gewährleisten.

Im folgenden wird ein Expressionssystem von HIV-1-Antigenen in E. coli beschrieben, das folgende Vorteile gegenüber der klassischen Antigengewinnung und Testentwicklung bietet: Die potentielle Infektiosität ist sowohl auf der Ebene der Produktion als auch der des Anwenders aufgehoben. Eine Kontamination durch Proteine aus den humanen Zellinien, die im Testansatz "unspezifische" Reaktionen hervorrufen, wird ausgeschlossen. Das rekombinante Expressionssystem ermöglicht eine hohe Konzentration relevanter Proteine (Epitope) im Testsystem, unter Einschluß der entsprechenden Vorläuferproteine, was bei der klassischen Virusreinigung nur schwer bzw. nicht zu erreichen ist. Durch Verwendung rekombinanter Antigene kann in ELISA-Testen unverdünntes Probematerial eingesetzt werden, was eine gute Handhabbarkeit des Tests gewährleistet.

**Herstellung rekombinanter Expressionsvektoren**

Als Basis für die Herstellung von Vektoren, die die Synthese von gp120 und/oder gp41 gewährleisten, diente das 3750 bp große Sall-Sstl-cDNA-Fragment des HTLVIII Isolates Stamm BH10, welches in den Vektor pUC18 (verdaut mit Sall und Sstl) kloniert worden war (pHIVenv). Ausgangsprodukt für die Expression für die core-Anteile war das ca. 1700 bp große Sstl-Bcll-cDNA Fragment, das in pUC19 (verdaut mit Sstl und BamHI) kloniert worden war (pHIV core). Die Angabe von Restriktionsschnittstellen und die Angabe von Basenpaaren (bp) beziehen sich im weiteren immer auf die publizierte Sequenz des HTLVIII BH10 Isolates (Ratner et al. (1985), Science 313,277-284). Da viele Bereiche der HIV-1-Proteine, ähnlich wie auch bestimmte Anteile von anderen viralen Proteinen, ziemlich instabil in E. coli sind und z.T. das Wachstum der Mikroorganismen hemmen (Crowl et al., Cell 41, 979-986 (1985)), wurden die DNA-Fragmente, die für die HIV-1-Hüllproteine oder core-Proteine codieren, dergestalt in Expressionsvektoren einligiert, daß die neuen rekombinierten Vektoren für Fusionsproteine codieren, wobei der Nicht-HIV-1-Anteil ein Fragment der E. coli β-Galactosidase darstellt. Dieser β-Gal-Anteil der produzierten Fusionsproteine schützt viele HIV-1-Proteinanteile vor Abbau in E. coli und ermöglicht eine einfache Reinigung der rekombinanten HIV-1-Proteine, während rekombinante gp160-Anteile, die mit dem $P_L$-Promotorsystem und ohne Fusion synthetisiert werden, schnell degradiert werden (Crowl et al, (1985), Cell 41, 979-986).

## Konstruktion von Vektoren zur Expression von HIV-Hüllproteinen

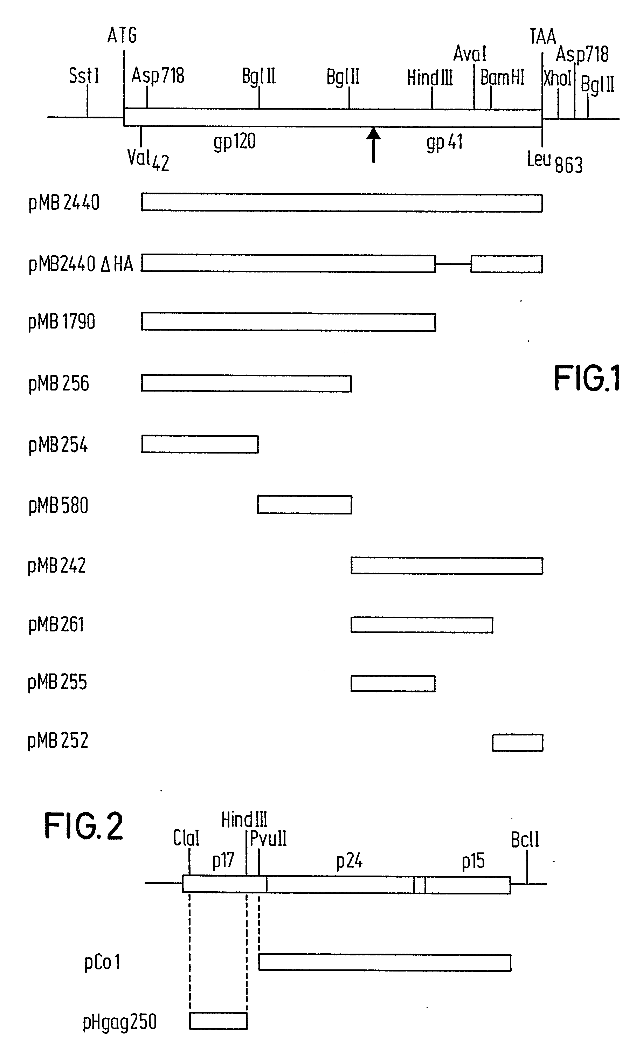

Da bekannt ist, daß hydrophobe Aminosäuresequenzen innerhalb eines Proteins die Expression desselben in E. coli negativ beeinflussen können und solche Proteine häufig instabil in Bakterien sind, wurde bei den Konstruktionen darauf verzichtet, die Präform des gp160 einschließlich der Signalsequenz zu exprimieren. Als größtes Produkt sollte ein Protein ohne die aminoterminale Signalsequenz synthetisiert werden, da diese im prozessierten gp160, dem gp120, fehlt und voraussichtlich in E. coli die Syntheserate negativ beeinflussen würde. Alle Vektorkonstruktionen wurden so vorgenommen, daß die HIV-1-Hüllproteinanteile im Leserahmen zum Aminoterminus der $\beta$-Galactosidase stehen, der durch das Plasmid pBD2 (Bröker, Gene Anal. Techn. 3 (1986), 53-57) oder Derivaten dieses Expressionsvektors codiert ist. Als Wirt für die Plasmidtransformationen diente E. coli BMH71-18 (lac pro)$_\Delta$ thi supE/F' lacI$^q$Z$^-$$\Delta$M15 pro$^+$, EMBO J. 1 (1982), 509-512). Die Methode der Klonierungsarbeiten sowie die Analyse der Expressionsprodukte beruht auf dem Handbuch "Molecular Cloning" von T. Maniatis et al., Cold Spring Harbor Laboratory, Cold Spring Harbor, New York (1982). Fig. 1 zeigt eine Übersicht über die Teile des Hüllproteins gp160, die mit Hilfe der pBD-Vektorserie in E. coli synthetisiert wurden.

### Beispiele

### pMB2440:

Die überstehenden Enden des 2571 bp großen Asp718 cDNA-Fragmentes aus pHIVenv von bp 5925-8496 wurden mit Polymerase I (Klenow-Fragment; PolI) in Gegenwart von Nukleotiden aufgefüllt und in pBD2 kloniert, das mit HindIII geschnitten und ebenfalls mit PolI behandelt worden war. Das neue Plasmid pMB2440 codiert somit für das HIV gp160 von Aminosäure Valin$_{42}$ bis Leucin$_{863}$, fusioniert an $\beta$-Gal$_{1-375}$.

### pMB2440$\Delta$HA:

Das Plasmid pMB2440$\Delta$HA wurde in mehreren Schritten konstruiert. Als erstes wurde pMB2440 mit XhoI linearisiert, mit PolI behandelt und religiert. Das neue Plasmid, das die XhoI-Stelle und gleichzeitig die AvaI-Stelle in Position bp8474 bis 8479 verloren hatte, aber noch die AvaI-Stelle in Position 7970-7975 behalten hatte, wurde pMB2440/6 benannt. Das Plasmit pMB2440/6 wurde anschließend mit HindIII und AvaI verdaut und das große DNA-Fragment von dem kleinen HindIII-AvaI-Fragment abgetrennt. Das große DNA-Fragment wurde mit PolI behandelt und religiert. Das so entstandene Plasmid pMB2440$\Delta$HA unterscheidet sich von pMB2440 darin, daß im gp41-Anteil des Fusionsproteins eine Deletion von Aminosäure Serin$_{647}$ bis Prolin$_{731}$ vorliegt.

### pMB1790

Das 1797bp große Asp718-HindIII cDNA-Fragment von bp5925-7722 wurde mit PolI behandelt und in pBD2 kloniert, das mit HindIII geschnitten und ebenfalls mit PolI behandelt worden war. Somit codiert das neue Plasmid pMB1790 für das gp160 von Aminosäure Valin$_{42}$ bis Serin$_{647}$. Im Vergleich zu dem Expressionsprodukt, das durch pMB2440 codiert ist, fehlen dem durch pMB1790 codierten Fusionsprotein die carboxyterminalen 216 Aminosäuren des gp41.

### pMB256

Um pMB256 zu erhalten, wurde pMB2440 mit XhoI linearisiert und anschließend mit BglII unvollständig verdaut. Die Reaktionsprodukte wurden auf einem 0,8 % Agarosegel aufgetrennt und das ca. 5070bp große DNA-Fragment isoliert. Anschließend wurde das DNA-Fragment mit PolI behandelt und religiert. Das neue Plasmit pMB256 hat die BglII Stelle von bp7198-7203 verloren, besitzt aber noch die BglII-Stelle bei Position bp6618 bis 6623 und codiert für ein Fusionsprotein, den den gp160-Anteil von Valin$_{42}$ bis Isoleucin$_{474}$ beinhaltet.

**pMB254:**

Um pMB254 zu erhalten, wurde pMB2440 mit XhoI linearisiert und anschließend mit BglII vollständig verdaut. Das 4490bp DNA-Fragment wurde isoliert, PolI behandelt und in Anwesenheit des DNA-Linkers 5´d (GCTTAATTAATTAAGC) 3´ religiert. Das durch pMB254 codierte Fusionsprotein umfaßt somit den gp120-Anteil von $Valin_{42}$ bis $Arginin_{280}$, ist also um 194 Aminosäuren kleiner als das durch pMB256 codierte Protein.

**pMB580:**

Um pMB580 zu erhalten, wurde das 580 bp BglII-Fragment aus pHIVenv, das für den gp120-Anteil von Aminosäure $Serin_{281}$ bis $Isoleucin_{474}$ codiert, mit PolI behandelt und in pBD2 ligiert, das mit HindIII linearisiert und mit PolI behandelt worden war.

**pMB242**

Das Plasmid pHIVenv wurde mit BglII verdaut und das ca. 1400bp große Fragment isoliert, mit PolI behandelt und anschließend in den Vektor pBD2IC20H ligiert, nachdem dieser mit NruI geschnitten worden war. Zuvor war pBD21C20H als Derivat von pBD2 zur Vereinfachung der Klonierungsarbeiten wie folgt konstruiert worden: Das ca. 60bp große BamI-HindIII Polylinker-Fragment aus pIC20H (Marsh et al., Gene 32, 481-485 (1984) war in pBD2, das mit BamHI und HindIII verdaut worden war, einligiert worden. Somit codiert pMB242 für ein Fusionsprotein mit einem gp160-Anteil von Aminosäure $Isoleucin_{474}$ bis $Leucin_{862}$.

**pMB261:**

Aus pMB242 wurde das ca. 860bp BamHI-Fragment isoliert und in die BamHI-Stelle von pBD21C20H ligiert. Das neue Plasmid pMB261 codiert für ein Fusionsprotein mit einem gp160-Anteil von $Isoleucin_{479}$ bis $Serin_{759}$. Das durch pMB261 codierte Fusionsprotein besitzt also 104 carboxyterminal gelegene Aminosäuren des gp41 weniger als das durch pMB242 codierte Fusionsprotein.

**pMB255:**

Der Vektor pMB242 wurde mit HindIII verdaut und das ca. 700 bp große DNA-Fragment abgetrennt. Das große DNA-Fragment wurde isoliert, PolI behandelt und religiert. Das neue Plasmid pMB255 codiert somit für ein Fusionsprotein mit einem Anteil von gp160 von Aminosäure $Isoleucin_{474}$ bis $Serin_{647}$.

**pMB252:**

Das ca. 430bp große BamHI-Fragment aus pHIVenv wurde isoliert, PolI behandelt und in pBD2IC20H ligiert, das zuvor mit SmaI linearisiert worden war. Das neue Plasmid pMB252 codiert für ein Fusionsprotein mit einem gp41-Anteil von Aminosäure $Serin_{759}$ bis $Leucin_{863}$.

Die Plasmide pMB2440, pMB2440ΔHA, pMB1790, pMB242, pMB261 und pMB255 codieren alle für HIV Hüllproteinanteile, die die Übergangssequenz vom gp120 zum gp41 besitzen und eignen sich somit für die Herstellung von Antigenen, wie sie in eukaryontischen Zellen wenn überhaupt nur in sehr geringem Ausmaß zu gewinnen sind.

**Konstruktion von Vektoren zur Expression von HIV core-Antigenen**

Zur Konstruktion von Vektoren zur Expression von core-Antigenen des HIV-1 wurde wie bei den oben beschriebenen Plasmiden zur Herstellung von HIV-Hüllproteinen Fusionierungen von core-Antigen-spezifischer cDNA an das E. coli lacZ Gen (codierend für β-Gal) vorgenommen (siehe Fig. 2).

**Beispiele**

**pCol:**

pHIV core wurde mit PvuII und XbaI verdaut und das ca. 1350bp cDNA-Fragment isoliert und in pBD2IC20H kloniert, das mit SmaI und XbaI verdaut worden war. Das neue Plasmid pCo1 codiert für den core-Anteil von $Asparaginsäure_{121}$ bis $Glutamin_{512}$, fusioniert an $\beta$-Galactosidase. Das Fusionsprotein umfaßt somit das gesamte p24 und p15 sowie die carboxyterminalen 13 Aminosäuren von p17.

**pHgag250**

Um pHgag250 zu erhalten, wurde das Plasmid pHIV-core mit ClaI linearisiert und mit PolI behandelt, anschließend mit HindIII nachgeschnitten, das ca. 270bp große DNA-Fragment isoliert und in das Plasmid pBD2FXa kloniert, das mit StuI und HindIII verdaut worden war. Das neue Plasmid pHgag250 codiert für den p17 Proteinanteil von Aminosäure $Arginin_{15}$ bis $Alanin_{100}$, fusioniert an $\beta$-Galactosidase. Der Vektor pBD2 FXa war zuvor wie folgt konstruiert worden: pBD2 wurde mit BamHI und HindIII verdaut und der folgende BamHI-HindIIIDNA-Linker eingebaut. Das neue Plasmid enthält somit singuläre Schnittstellen für BamHI, StuI und HindIII 3′ endig vom lacZ Genfragment.

**Verwendung der rekombinanten HIV-1-Antigene als diagnostisches Mittel**

Die vorstehend beschriebenen in E. coli synthetisierten HIV-1-Antigene wurden alle spezifisch von HIV-1-Antikörper positiven Patientenseren erkannt. Der Nachweis dieser Immunreaktionen wurde wie folgt durchgeführt: E. coli BMH71-18-Zellen, die das Plasmid pBD2 (Kontrolle) oder die oben beschriebenen HIV-Expressionsvektoren enthalten, wurden über Nacht in 5 ml M9-Medium, das mit 0,05 ml LB-Medium supplementiert worden war, angezogen.

Die Medienrezepte und Wachstumsbedingungen sind aufgeführt in "Molecular Cloning" (Maniatis, T., et al., a.a.O. ). Nach ca. 15 bis 18 Stunden wurden die Zellen abzentrifugiert und in 5 ml frisches M9-Medium resuspendiert. Nach zwei Stunden Wachstum wurde IPTG in einer Endkonzentration von 1 mM zugesetzt, um den lac-Promotor der Expressionsvektoren zu induzieren. Nach weiteren zehn Minuten wurden ca. 10-30 $\mu$ci $^{35}$S -L-Methionin mit einer spezifischen Aktivität von 800 Ci/mmol der jeweiligen Kultur zugesetzt und nach weiteren 15 Minuten wurden die Zellen durch Zentrifugation geerntet. Die Zellen wurden in 0,1 ml Lysozymlösung (10 mg Lysozym/ml in 0,25 M Tris-HCl, pH 8,0) aufgenommen und 0,03 ml 0,2 M EDTA-Lösung zugesetzt. Nach einigen Minuten wurden 0,7 ml Aufschlußpuffer IP3 (20 mM Tris -HCl pH 8,0 100 mM NaCl, 1 mM EDTA, 1 % Nonidet P-40, 1 % Natriumdesoxycholat, 0,1 % SDS) zugesetzt und die Bakteriensuspension dreimal in flüssigem Stickstoff eingefroren und im Wasserbad bei 37°C aufgetaut. Anschließend wurde das Bakterienlysat 15 Minuten in der Eppendorf-Tischzentrifuge 5414 zentrifugiert und das Sediment verworfen. 0,05 ml vom Überstand wurden mit 0,05 ml cracking-Puffer fünf Minuten bei 100°C erhitzt und zur Analyse der Gesamtproteine des Zellextraktes auf ein SDS-Polyacrylamid-Gel aufgetrennt. Der cracking-Puffer wurde wie folgt zusammengesetzt: 5 ml Tris-HCl pH 6,8 werden gemischt mit 10 ml 10 % SDS, 1 ml 0,2 M EDTA, 1 ml Merkaptoäthanol, 15 ml Glycerin, 10 ml Bromphenolblau und mit $H_2O$ auf 100 ml aufgefüllt.

0,2 ml des Überstandes wurden mit 0,02 bis 0,05 ml Humanseren versetzt und zwei Stunden bei 4°C inkubiert. Danach wurden 0,2 ml einer Protein A Sepharose-Suspension (30 mg Protein A Sepharose CL-4B, Pharmacia/ml IP3-Puffer) hinzugefügt und der Ansatz für weitere zwei Stunden bei 4°C bei leichtem Schütteln inkubiert. Die Suspension wurde 30 Sekunden in der Eppendorfzentrifuge 5414 abzentrifugiert und das Sediment in folgenden Puffern gewaschen: erstens -IP2-Puffer (20 mM Tris-HCl pH 8,0, 100 mM

6

NaCl, 1mM EDTA, 1 % NP-40, 1 % Natriumdesoxycholat, 0,1 % SDS, 2 % Rinderserumalbumin), zweitens - IP2-Puffer mit 1 M NaCl statt 0,1 M NaCl, drittens - IP3-Puffer, viertens - IP1-Puffer (20 mM Tris HCl pH 8,0 100 mM NaCl, 1 mM EDTA, 1 % NP40). Das Sediment wurde anschließend mit 0,2 ml cracking-Puffer versetzt und fünf Minuten bei 100°C erhitzt. Jeweils 0,01 - 0,05 ml dieser Immunpräzipitationslösung bzw. des Gesamtproteinextraktes wurden auf ein 9 % SDS-Polyacrylamidgel aufgetragen und die Proteine bei 150 V innerhalb von neun Stunden aufgetrennt. Anschließend wurde das Gel zehn Minuten in Enlighteninglösung (NEN Research Products) inkubiert und getrocknet. Das getrocknete Gel wurde 24 bis 70 Stunden bei - 70°C auf einem Röntgenfilm (Kodak XAR 5) exponiert und entwickelt. Bei Verwendung von anti HIV-I-positiven humanen Seren wurde im Gegensatz zu Seren von gesunden Probanden jedes rekombinante HIV-I-Fusionsprotein, nicht aber das β-Galactosidase Fragment, das durch pBD2 codiert wird, spezifisch erkannt. Somit konnte gezeigt werden, daß AIDS-Patientenseren gegen alle oben aufgeführten Antigene der Hüllproteine und der core-Proteine, die in E. coli exprimiert worden sind, Antikörper gebildet haben. Die antigenen Determinanten der HIV-I-Proteine, die im Menschen die spezifische Antikörperproduktion induzieren, sind offensichtlich somit über den jeweiligen Proteinen verteilt und nicht, wie z.B. im Fall von Hepatitis B-Virus auf einen distinkten Abschnitt (core-Protein) beschränkt.

Die Intensität der Banden auf dem Röntgenfilm war unterschiedlich, je nachdem, welches Fusionsprotein zur Immunpräzipitation mit einem bestimmten Patientenserum eingesetzt wurde. Bezogen auf Fusionsproteine, die Teile des gp41 beinhalten, wurden am besten HIV-1-Proteinsequenzen immunpräzipitiert, die durch pMB242, pMB261 und pMB255 codiert sind, während das durch pMB252 codierte Protein zwar spezifisch erkannt wird, aber relativ schwächer reagiert.

Unter den core-Proteinen wird insbesondere das durch pCol codierte Fusionsprotein sehr gut von Patientenseren erkannt. Außer Seren von AIDS-Patienten wurden in der Immunpräzipitation auch Seren von Pre-AIDS-Individuen getestet, die in üblichen Western Blot-Analysen schwach und nur mit einigen HIV-1-Proteinen scheinbar spezifisch reagieren. Es zeigte sich, daß einige Pre-AIDS-Seren, z.B. gegen das pMB 2440-Protein, pMB2440ΔHA-Protein, pMB242-Protein und pMB261-Protein reagierten, nicht aber gegen das pMB256-Protein. Somit erkannten diese Seren rekombinante gp41-Anteile, nicht aber gp120-Sequenzen.

Weiterhin reagierten einige Pre-AIDS-Seren z.T. gegen pCol-Protein, nicht aber gegen das pHgag250-Protein. Eine Erklärung für diesen Befund könnte sein, daß in Seren von Pre-AIDS-Individuen teilweise schon Antikörper gegen p24 und/oder p15 vorhanden sind, nicht aber gegen p17. Die ermittelte Variabilität in Bezug auf die Erkennung verschiedener HIV-1-Proteine zum Antikörpernachweis dokumentiert die Notwendigkeit, eine möglichst breite Palette von rekombinanten HIV-1-Proteinen zur Diagnostik zu verwenden. Andererseits haben sich Fusionsproteine, codiert durch pMB2440, pMB2440ΔHA, pMB1790, pMB261 (Hüllproteine) und pCol (core-Antigene) als besonders geeignet herausgestellt, um HIV-1-Antikörper in Humanseren nachzuweisen. Im weiteren eignet sich die beschriebene Palette von exprimierten Antigenen des gp160, p17, p24 und p15 zur Spezifizierung der Antikörper von Patienten und kann für epidemiolgoische Studien hilfreich sein.

Als weiteres System zum Antikörpernachweis in humanen Seren wurden Western Blot-Analysen nach Burnette (Analyt. Biochem. 112, 195 bis 203 (1981)) vorgenommen. Hierzu wurden Rohextrakte von E. coli mit den oben beschriebenen Expressionsvektoren auf einem SDS-Polyacrylamidgel aufgetrennt, die Proteine auf Nitrocellulose transferiert und anschließend mit Patientenseren inkubiert. Als Konjugat dienten anti-Human IgG-Antikörper aus Kaninchen, die mit alkalischer Phosphatase gekoppelt worden waren. In diesen Western Blot-Analysen zeigte sich ebenfalls, daß alle AIDS-Patienten-Seren spezifisch mit den HIV-1-Fusionsproteinen, nicht aber mit der β-Galactosidase reagierten. Als drittes System zum Nachweis von HIV-1-Antikörpern kann der ELISA durchgeführt werden. Hierzu müssen die entsprechenden rekombinanten Antigene in größeren Mengen hergestellt, gereinigt und auf Mikrotiterplatten beschichtet werden. Aufgrund ihrer Stabilität in E. coli ließen sich insbesondere pMB1790, pMB256, pMB580, pMB252, pCol und pHgag250 Protein produzieren. Die Reinigung kann wegen des β-Galactosidase-Anteils, der in allen Fusionsproteinen vorhanden ist, schnell und einfach wie folgt durchgeführt werden: 1 l LB-Medium wird mit 100 ml einer über Nacht-Vorkultur angeimpft. Nach 1,5 Stunden wird mit IPTG in einer Endkonzentration von 1 mM der lac-Promotor induziert und die Zellen werden nach drei bis fünf Stunden geerntet, in 10mM Tris-HClpH 7,5 gewaschen und anschließend in ca.15 ml 20 mM Tris-HCl pH 7,5 resuspendiert. Die Zellen werden mit Ultraschall aufgeschlossen und die Zelltrümmer einschließlich der unlöslichen Fusionsproteine abzentrifugiert. Anschließend wird das Sediment in 1 M Harnstoff-Lösung aufgenommen und 30 Minuten bei Raumtemperatur inkubiert. Nach Zentrifugation (Sorvall-Zentrifuge Rotor SS34, 20 Minuten, 10.000 UpM) wird das Sediment in 10M Harnstofflösung aufgenommen und 1 Stunde bei Raumtemperatur inkubiert. Nach erneuter hochtouriger Zentrifugation befinden sich die Fusionsproteine größtenteils im Überstand. Der Überstand wird ausgiebig gegen 20 mM TrisHCl pH 7,5 dialysiert und die Proteinlösung bei -20°C eingefroren. Wenn man die Lösung anschließend auftaut und wiederum hochtourig abzentrifugiert, verblei-

7

ben die Fusionsproteine vorwiegend im Überstand, während andere E. coli Proteine, insbesondere Membranproteine, sich im Sediment befinden. Die so gereinigten Fusionsproteine können entweder weiter gereinigt werden oder sind direkt einsetzbar als Antigen in Western Blot oder ELISA-Analysen.

Während in Western Blots die Reaktivität der rekombinanten core-Proteine etwa den gereinigten Virusproteinen entsprach, waren die rekombinanten Hüllproteine dem konventionellen System in der Intensität der Immunreaktion überlegen. Rekombinante Proteine aus E. coli sind nicht glykosyliert. Dies erwies sich für die Gelelektrophorese und Proteintransfer beim Immunoblot mit rekombinanten Hüllproteinen als Vorteil, da scharf umgrenzte Proteinbanden entstehen. Der Test auf Antikörper gegen die HIV-Hüllproteine ist für die Western Blot-Diagnostik relevant, da anti gp120/gp41 konsistent vorkommt, während core-Proteine mit Fortschreiten der Erkrankung häufig nicht mehr nachgewiesen werden können. Ein besonderer Vorteil des hier beschriebenen Expressionssystems liegt darin, daß reichlich Fusionsproteine von hoher Reaktivität entstehen. Somit kann man auch, ohne die Proteine zu reinigen, gesamte Extrakte von E. coli in niedriger Konzentration für Western Blots einsetzen. Im allgemeinen war die Reaktion positiver Seren mit den Fusionsproteinen so stark, daß mögliche Begleitreaktivität mit dem $\beta$-Galactosidaseanteil nicht ins Gewicht fiel.

Die Fusionsproteine können aber nicht nur direkt als Antigen zur Diagnostik eingesetzt werden, sondern auch indirekt zur Produktion von polyklonalen monospezifischen Seren und monoklonalen Antikörpern. Beispielhaft wurden Meerschweinchen, Kaninchen und Ziegen mit pMB170-Protein, pMB252-Protein, pHgag250-Protein und pCol-Protein viermal mit je ca. 100 μg bzw. 500 μg (Ziegen) immunisiert, wobei die Fusionsproteine nach dem obigen Schema gereinigt worden waren. In Western blot-Analysen zeigte sich, daß Seren der Tiere vor Immunisierung nicht mit HIV-1-Proteinen reagierten, wohl aber Seren der betreffenden immunisierten Tiere, wobei teilweise (z.B. pCol-Protein) schon nach der ersten Immunisierung eine positive Reaktion selbst bei einer 1:500 Verdünnung vorhanden war. Diese Ergebnisse zeigen, daß die in E. coli exprimierten antigenen Epitope der HIV-1-Proteinsequenzen in der Lage sind, in Meerschweinchen, Kaninchen und Ziegen Antikörper zu erzeugen, die mit authentischen Virusproteinen reagieren. Im Falle von einer Immunisierung mit dem pMB252-Protein reagieren die produzierten Antikörper nicht nur mit dem gp41 des HIV-1, sondern auch mit dem Vorläuferprotein gp160. Zusätzlich zur Western Blot-Analytik wurden die HIV-1-Antikörper nach Immunisierung von Versuchstieren im ELISA bestimmt. Die Immunogenität der rekombinanten HIV-1-Proteine ist beispielhaft in der Tabelle 1 dokumentiert.

Da beträchtliche Kreuzreaktivität von HIV-2 spezifischen Antikörpern zu den oben beschriebenen HIV-1 Antigenen besteht, können diese auch bei der Diagnostik von HIV-2 entsprechend Verwendung finden.

Antikörper, die mit Hilfe der hier beschriebenen Fusionsproteine hergestellt worden sind, lassen sich verwenden zum Antigennachweis, z.B. als Bestandteil eines ELISA, sowie zum Isolieren von HIV-Antigenen.

## Tabelle 1

**Bestimmung des Antikörpertiters in Meerschweinchenserum nach Immunisierung mit rekombinanten HIV-1-Antigenen**

| Antigen | Meerschwein-Nr. | Immunisierung am Tag | Serum v. Tag | Verdünnung d. Serums | Extinktion* |
|---|---|---|---|---|---|
| pMB1790 | 7 | 0 | 0 | 1 : 100 | 0,20 |
| | | 14 | 23 | | 0,27 |
| | | 28 | 36 | | 0,70 |
| | | 51 | 58 | | 0,79 |
| | 8 | 0 | 0 | 1 : 100 | 0,07 |
| | | 14 | 23 | | 0,61 |
| | | 28 | 36 | | 0,79 |
| | | 51 | 58 | | 1,68 |
| | 9 | 0 | 0 | 1 : 100 | 0,02 |
| | | 14 | 23 | | 0,22 |
| | | 28 | 36 | | 1,00 |
| | | 51 | 58 | | 1,13 |
| pHgag250 | 13 | 0 | 0 | 1 : 100 | 0,03 |
| | | 14 | 23 | | 0,83 |
| | | 28 | 36 | | 2,50 |
| | 14 | 0 | 0 | | 0,02 |
| | | 14 | 23 | | 1,09 |
| | | 28 | 36 | | 1,95 |

**Fortsetzung Tabelle 1**

| Antigen | Meerschwein-Nr. | Immunisierung am Tag | Serum v. Tag | Verdünnung d. Serums | Extinktion[*] |
|---|---|---|---|---|---|
| pCol | 16 | 0 | 0 | 1 : 100 | 0,018 |
|  |  | 14 | 23 |  | 2,50 |
|  |  | 28 | 36 |  | 2,50 |
|  |  | 51 | 58 | 1 : 80000 | 1,23 |
|  | 17 | 0 | 0 | 1 : 100 | 0,02 |
|  |  | 14 | 23 |  | 2,50 |
|  |  | 28 | 36 |  | 2,50 |
|  |  | 51 | 58 | 1 : 80000 | 1,34 |
| pMB252 | 21 | 0 | 0 |  | 0,02 |
|  |  | 19 | 25 |  | 0,15 |
|  |  | 33 | 56 |  | 0,43 |

\* Extinktion gemessen bei 492 mm im indirekten ELISA
(Einsatz von Anti-Meerschweinimmunglobulinperoxidase-
Konjugat und o-Phenylendiamin/$H_2O_2$)

**Ansprüche**

1. Rekombinante unprozessierte HIV-1-Proteine.

2. Rekombinantes unprozessiertes Hüllprotein gp160 von HIV-1 oder Teile davon.

3. Fusionsprotein, gekennzeichnet durch rekombinantes Hüllprotein gp160 oder Teile davon nach Anspruch 2 als Fusionspartner.

4. Rekombinantes Hüllprotein gp160 von HIV-1 oder Teile davon nach Anspruch 2, dadurch gekennzeichnet, daß die HIV-1-spezifischen Proteinanteile an E. coli β-Galactosidase oder Teile derselben fusioniert sind.

5. Rekombinantes unprozessiertes core-Protein von HIV-1, p24, p17 und p15 umfassend.

6. Fusionsprotein, gekennzeichnet durch rekombinantes core-Protein oder Teile davon nach Anspruch 5 als Fusionspartner.

7. Fusionsprotein, gekennzeichnet durch rekombinantes core-Protein p24 oder Teile davon als Fusionspartner.

8. Fusionsprotein, gekennzeichnet durch rekombinantes core-Protein p17 oder Teile davon als Fusionspartner.

9. Fusionsprotein, gekennzeichnet durch rekombinantes core-Protein p15 oder Teile davon als Fusionspartner.

10. Rekombinantes fusioniertes core-Protein nach Anspruch 6 bis 9, dadurch gekennzeichnet, daß ein Fusionsanteil aus E. coli β-Galactosidase oder Teilen derselben besteht.

11. Verwendung rekombinanter HIV-1 Proteine nach Anspruch 1 bis 10 für diagnostische Zwecke und als Bestandteil einer Vakzine.

12. Verwendung rekombinanter HIV-1 Proteine nach Anspruch 1 bis 10 zur Herstellung spezifischer poly- und monoklonaler Antikörper.

13. Verwendung von anti-HIV-1-Antikörpern nach Anspruch 12 für diagnostische Zwecke und als Bestandteil eines Passiv-Impfstoffs.

14. Verwendung von anti-HIV-1-Antikörpern nach Anspruch 12 zum Nachweis von HIV-1 Antigen und Viruspartikeln.

Patentansprüche für den folgenden Vertragsstaat.

1. Verfahren zur Herstellung rekombinanter unprozessierter HIV-1-Proteine oder Teile davon, dadurch gekennzeichnet, daß die dafür kodierenden DNA-Fragmente in Vektoren einligiert, E. coli-Zellen mit diesen Vektoren transformiert und in den Transformanten exprimiert werden.

2. Verfahren nach Anspruch 1, dadurch gekennzeichnet, daß die rekombinanten unprozessierten HIV-Proteine oder Teile davon als Fusionsproteine mit E. coli Proteinen exprimiert werden.

3. Verfahren nach Anspruch 2, dadurch gekennzeichnet, daß der Fusionspartner aus E. coli die $\beta$-Galactosidase ist oder Teile davon.

4. Verfahren nach Anspruch 1 bis 3, dadurch gekennzeichnet, daß das rekombinante Hüllprotein gp 160 oder Teile davon exprimiert wird.

5. Verfahren nach Anspruch 1 bis 3, dadurch gekennzeichnet, daß rekombinante unprozessierte core-Proteine aus p24, p17 und/oder p15 oder Teile davon exprimiert werden.

6. Verfahren nach Anspruch 1 bis 5 dadurch gekennzeichnet, daß mit Hilfe der erhaltenen HIV-Proteine spezifische poly- oder monoklonale Antikörper hergestellt werden.

7. Verfahren nach Anspruch 7, dadurch gekennzeichnet, daß die erhaltenen spezifischen Antikörper für den Nachweis von HIV-1 Antigenen, als Teil eines diagnostischen Kits, als Bestandteil einer Vakzine oder als Bestandteil eines Passiv-Impfstoffs eingesetzt werden.

FIG.1

FIG.2